(19) Europäisches Patentamt
European Patent Office
Office européen des brevets

(11) Veröffentlichungsnummer: **0 297 352 B1**

(12) # EUROPÄISCHE PATENTSCHRIFT

(45) Veröffentlichungstag der Patentschrift: **02.12.92**

(51) Int. Cl.[5]: **C07D 249/08**, C07D 233/60, A01N 43/56, A01N 43/653

(21) Anmeldenummer: **88109522.8**

(22) Anmeldetag: **15.06.88**

Die Akte enthält technische Angaben, die nach dem Eingang der Anmeldung eingereicht wurden und die nicht in dieser Patentschrift enthalten sind.

(54) **(Azolyl-vinyl)-phenol-alkenylether.**

(30) Priorität: **27.06.87 DE 3721336**
**15.04.88 DE 3812483**

(43) Veröffentlichungstag der Anmeldung:
**04.01.89 Patentblatt 89/01**

(45) Bekanntmachung des Hinweises auf die Patenterteilung:
**02.12.92 Patentblatt 92/49**

(84) Benannte Vertragsstaaten:
**BE CH DE ES FR GB GR IT LI NL**

(56) Entgegenhaltungen:
**EP-A- 0 149 976** **EP-A- 0 162 359**
**EP-A- 0 255 243** **DE-A- 2 634 511**
**DE-A- 2 656 728** **DE-A- 3 021 467**

**CHEMICAL ABSTRACTS, Band 103, Nr. 23, 9. Dezember 1985, Seite 692, Spalte 2, Zusammenfassung-nr. 196102x, Columbus, Ohio, US; KUMIAI CHEM. IND. Co. Ltd.: "Fungicidal (pyridylimidoyl)imidazoles"**

**CHEMICAL ABSTRACTS, Band 108, Nr. 7, 15. Februar 1988, Seite 26, Spalte 2, Zusammenfassung-nr. 51138g, Columbus, Ohio, US; KATAOKA T. et al.: "Ouantitative structure-activity relationships of antifungal 1-[1-substituted phenyl)vinyl]imidazoles"**

(73) Patentinhaber: **BAYER AG**

**W-5090 Leverkusen 1 Bayerwerk(DE)**

(72) Erfinder: **Schade, Gerold, Dr.**
**Hahnenweg 8**
**W-5000 Köln 80(DE)**
Erfinder: **Brandes, Wilhelm, Dr.**
**Eichendorffstrasse 3**
**W-5653 Leichlingen 1(DE)**
Erfinder: **Hänssler, Gerd, Dr.**
**Am Arenzberg 58a**
**W-5090 Leverkusen 31(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Die vorliegende Erfindung betrifft neue (Azolyl-vinyl)-phenol-alkenylether, ein Verfahren zu deren Herstellung und deren Verwendung als Mikrobizide im Pflanzenschutz und im Materialschutz.

Es ist bereits bekannt, daß sich zahlreiche Benzylimidazol-Derivate zur Bekämpfung von Pilzen und Bakterien verwenden lassen (vgl. DE-OS 3 021 467, DE-OS 3 500 503 und EP-OS 0 162 359). So lassen sich zum Beispiel 1-(1-[2-(Thien-2-yl-methoxy)-phenyl]-vinyl)-imidazol, 1-(1-[2-(Thien-2-yl-methoxy)-phenyl]-2,2-dimethyl-vinyl)-imidazol und 1-(1-[2-(3-Chlorphenoxy-methoxy)-phenyl]-vinyl)-imidazol als Fungizide gegen phytopathogene Pilze einsetzen. Bei niedrigen Aufwandmengen läßt die Wirksamkeit dieser Stoffe allerdings in manchen Fällen zu wünschen übrig.

Es wurde nun neue (Azolyl-vinyl)-phenol-alkenylether der Formel

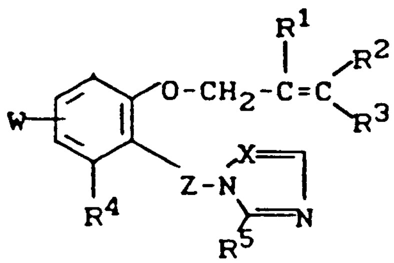

(I)

in welcher

$R^1$ für Wasserstoff, Fluor, Chlor, Brom oder Alkyl mit 1 bis 4 Kohlenstoffatomen,

$R^2$ für Wasserstoff, Fluor, Chlor, Brom, Alkyl mit 1 bis 4 Kohlenstoffatomen, Alkenyl mit 2 bis 4 Kohlenstoffatomen, wobei jeder dieser Alkenyl-Reste substituiert sein kann durch gegebenenfalls substituiertes Aryl, oder für gegebenenfalls einfach bis dreifach, gleichartig oder verschieden durch Alkyl mit 1 bis 4 Kohlenstoffatomen, Halogen und/oder Halogenalkyl mit 1 oder 2 Kohlenstoffatomen und 1 bis 5 Halogenatomen substituiertes Phenyl, oder

$R^1$ und $R^2$ gemeinsam für eine C-C-Bindung,

$R^3$ für Wasserstoff, Fluor, Chlor, Brom, Iod, Alkyl mit 1 bis 12 Kohlenstoffatomen oder für gegebenenfalls einfach bis dreifach, gleichartig oder verschieden durch Alkyl mit 1 bis 4 Kohlenstoffatomen, Halogen und/oder Halogenalkyl mit 1 oder 2 Kohlenstoffatomen und 1 bis 5 Halogenatomen substituiertes Phenyl,

$R^4$ für Wasserstoff,

$R^5$ für Wasserstoff oder Alkyl mit 1 bis 4 Kohlenstoffatomen,

W für Wasserstoff, Fluor, Chlor oder Brom,

X für Stickstoff oder eine CH-Gruppe und

Z für die Gruppierungen

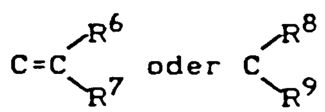

oder ,

worin

$R^6$ für gegebenenfalls einfach bis dreifach, gleichartig oder verschieden durch Alkyl mit 1 bis 4 Kohlenstoffatomen, Halogen und/oder Halogenalkyl mit 1 oder 2 Kohlenstoffatomen und 1 bis 5 Halogenatomen substituiertes Phenyl steht oder

$R^6$ gemeinsam mit $R^4$ für die Gruppierung

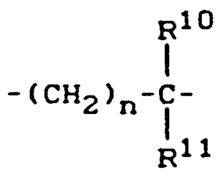

steht,

| | |
|---|---|
| $R^7$ | für Wasserstoff oder Alkyl mit 1 bis 4 Kohlenstoffatomen steht, |
| $R^8$ | für Wasserstoff oder Alkyl mit 1 bis 4 Kohlenstoffatomen steht, |
| $R^9$ | für Wasserstoff oder Alkyl mit 1 bis 4 Kohlenstoffatomen steht, |
| $R^{10}$ | für Wasserstoff oder Alkyl mit 1 bis 4 Kohlenstoffatomen steht, |
| $R^{11}$ | für Wasserstoff oder Alkyl mit 1 bis 4 Kohlenstoffatomen steht und |
| n | für die Zahlen 0, 1 oder 2 steht |

sowie deren Säureadditions-Salze und Metallsalz-Komplexe gefunden.

Weiterhin wurde gefunden, daß man (Azolyl-vinyl)-phenolalkenylether der Formel (I) sowie deren Säureadditions-Salze und Metallsalz-Komplexe erhält, wenn man Phenol-Derivate der Formel

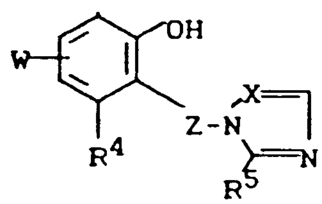

(II)

in welcher

| | |
|---|---|
| $R^4$, $R^5$, W, X und Z | die oben angegebene Bedeutung haben, |

mit Basen gegebenenfalls in Gegenwart eines Verdünnungsmittels umsetzt und die dabei entstehenden Phenolate der Formel

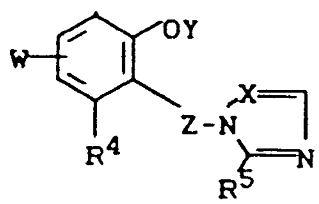

(II-a)

in welcher

| | |
|---|---|
| $R^4$, $R^5$, W, X und Z | die oben angegebene Bedeutung haben und |
| Y | für einen Baserest steht, |

mit Halogenverbindungen der Formel

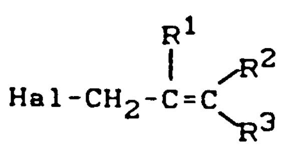

(III)

in welcher

| | |
|---|---|
| $R^1$, $R^2$ und $R^3$ | die oben angegebene Bedeutung haben und |
| Hal | für Chlor, Brom oder Iod steht, |

in Gegenwart eines Verdünnungsmittels sowie gegebenenfalls in Gegenwart eines Säurebindemittels umsetzt und gegebenenfalls anschließend an die so erhaltenen Verbindungen der Formel (I) eine Säure

oder ein Metallsalz addiert.

Außerdem wurde gefunden, daß die neuen (Azolyl-vinyl)-phenol-alkenylether der Formel (I) sowie deren Säureadditions-Salze und Metallsalz-Komplexe sehr gute mikrobizide Eigenschaften besitzen und sowohl im Pflanzenschutz als auch im Materialschutz eingesetzt werden können.

Überraschenderweise zeigen die erfindungsgemäßen Stoffe bei der Bekämpfung von phytopathogenen Pilzen eine deutlich bessere Wirksamkeit als 1-(1-[2-(Thien-2-yl-methoxy)-phenyl]-vinyl)-imidazol, 1-(1-[2-Thien-2-yl-methoxy)-phenyl]-2,2-dimethyl-vinyl)-imidazol und 1-(1-[2-(3-Chlorphenoxy-methoxy)-phenyl]-vinyl)-imidazol, welches konstitutionell ähnliche vorbekannte Verbindungen gleicher Wirkungsrichtung sind.

Die erfindungsgemäßen (Azolyl-vinyl)-phenol-alkenylether sind durch die Formel (I) allgemein definiert.

Besonders bevorzugt sind diejenigen Verbindungen der Formel (I), in denen

$R^1$ für Wasserstoff, Fluor, Chlor, Brom, Methyl, Ethyl, n-Propyl, Isopropyl oder n-Butyl steht,

$R^2$ für Wasserstoff, Fluor, Chlor, Brom, Methyl, Ethyl, n-Propyl, Isopropyl, n-Butyl, Vinyl, Propenyl oder Styryl steht, oder für gegebenenfalls einfach bis dreifach, gleichartig oder verschieden durch Methyl, Ethyl, Flour, Chlor, Brom und/oder Trifluormethyl substituiertes Phenyl steht oder

$R^1$ und $R^2$ gemeinsam eine C-C-Bindung stehen,

$R^3$ für Wasserstoff, Fluor, Chlor, Brom, Iod, Alkyl mit 1 bis 10 Kohlenstoffatomen oder für gegebenenfalls einfach bis dreifach, gleichartig oder verschieden durch Methyl, Ethyl, Fluor, Chlor und/oder Trifluormethyl substituiertes Phenyl steht,

$R^4$ für Wasserstoff steht,

$R^5$ für Wasserstoff, Methyl oder Ethyl steht,

W für Wasserstoff oder Chlor steht,

X für Stickstoff oder eine CH-Gruppe steht und

Z für die Gruppierungen

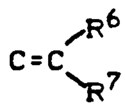 oder 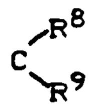

steht, worin

$R^6$ für gegebenenfalls einfach bis dreifach, gleichartig oder verschieden durch Methyl, Ethyl, Fluor, Chlor und/oder Trifluormethyl substituiertes Phenyl steht oder

$R^6$ gemeinsam mit $R^4$ für die Gruppierung

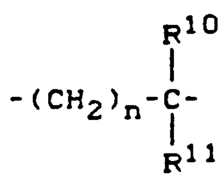

steht,

$R^7$ für Wasserstoff, Methyl, Ethyl, n-Propyl, Isopropyl oder n-Butyl steht,

$R^8$ für Wasserstoff, Methyl, Ethyl, n-Propyl, Isopropyl oder n-Butyl steht,

$R^9$ für Wasserstoff, Methyl, Ethyl, n-Propyl, Isopropyl oder n-Butyl steht,

$R^{10}$ für Wasserstoff oder Methyl steht,

$R^{11}$ für Wasserstoff oder Methyl steht und

n für die Zahlen 0 oder 1 steht.

Bevorzugte erfindungsgemäße Verbindungen sind auch Additionsprodukte aus Säuren und diejenigen (Azolyl-vinyl)-phenol-alkenylethern der Formel (I), in denen $R^1$, $R^2$, $R^3$, $R^4$, $R^5$, W, X und Z die Bedeutungen haben, die bereits vorzugsweise für diese Reste genannt wurden.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure

und Milchsäure, sowie Sulfonsäuren, wie z.B. p-Toluolsulfonsäure und 1,5-Naphthalindisulfonsäure.

Außerdem bevorzugte erfindungsgemäße Verbindungen sind Additionsprodukte aus Salzen von Metallen der II. bis IV. Haupt- und der I. und II. sowie IV. bis VIII. Nebengruppe des Periodensystems der Elemente und denjenigen (Azolyl-vinyl)-phenol-alkenylethern der Formel (I), in denen $R^1$, $R^2$, $R^3$, $R^4$, $R^5$, W, X und Z die Bedeutungen heben, die bereits vorzugsweise für diese Reste genannt wurden.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Die erfindungsgemäßen Stoffe können in Form von cis- und trans-Isomeren vorliegen, und zwar sowohl bezüglich der Substituenten an der Alkenylgruppe als auch für den Fall, daß Z für

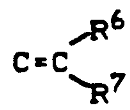

steht, sofern $R^6$ und $R^7$ verschieden sind. Die Erfindung betrifft sowohl die reinen cis- und trans-Isomeren als auch deren Gemische.

Verwendet man 1-[1-(2-Hydroxyphenyl)-vinyl]-imidazol als Ausgangsstoff, Natriumhydroxid als Base und 1-Chlor-2,3-dimethyl-but-2-en als Reaktionskomponente, so kann der Verlauf des erfindungsgemäßen Verfahrens durch das folgende Formelschema veranschaulicht werden:

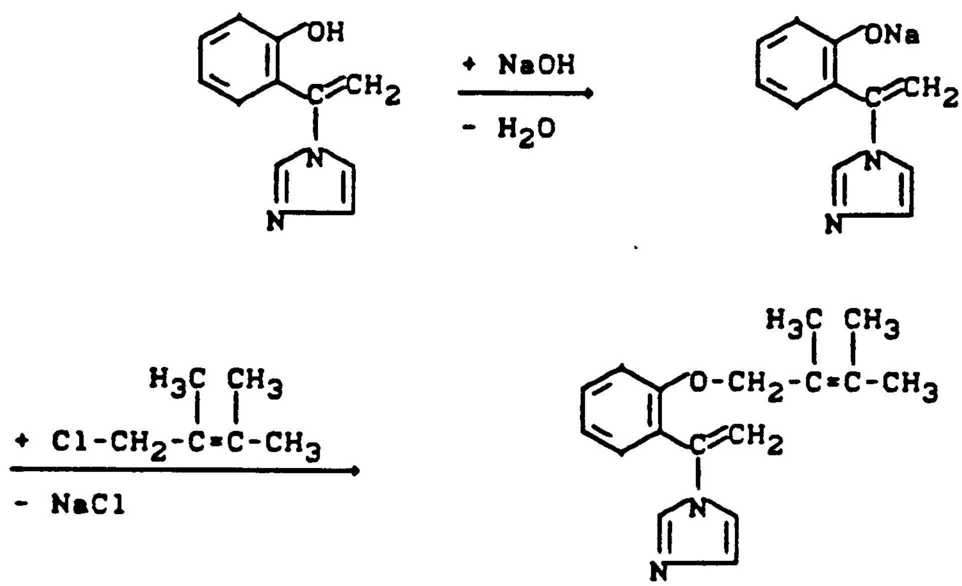


Die bei der Durchführung des erfindungsgemäßen Verfahrens als Ausgangsstoffe benötigten Phenol-Derivate sind durch die Formel (II) allgemein definiert. In dieser Formel haben $R^4$, $R^5$, W, X und Z vorzugsweise diejenigen Bedeutungen, die bereits im Zusammenhang mit der Beschreibung der erfindungsgemäßen Stoffe der Formel (I) vorzugsweise für diese Reste genannt wurden.

Die Phenol-Derivate der Formel (II) sind bekannt oder lassen sich nach prinzipiell bekannten Verfahren herstellen (vgl. J. Med. Chem. 27, 1142 (1984) und DE-OS 3 021 467).

Als Basen kommen bei der Durchführung des erfindungsgemäßen Verfahrens alle für derartige Umsetzungen üblichen starken Basen in Betracht. Vorzugsweise verwendbar sind Alkalimetallhydroxide, Alkalimetallamide, Alkalimetallalkoholate, Alkalimetallhydride, quarternäre Ammoniumhydroxide oder Phosphoniumhydroxide. Besonders bevorzugt sind Natriummethylat, Kalium-tert.-butylat, Natrium-amid, Natrium-hydrid und Tetramethylammonium-hydroxid. Demgemäß steht Y in der Formel (II-a) vorzugsweise

für ein Alkalimetall-kation, wie ein Natrium- oder Kalium-kation, oder für ein quarternäres Ammonium- oder Phosphonium-kation.

Als Verdünnungsmittel kommen bei der Durchführung der ersten Stufe des erfindungsgemäßen Verfahrens alle für derartige Umsetzungen üblichen organischen Solventien in Frage. Vorzugsweise verwendbar sind Alkohole, wie Methanol, Ethanol, n-Propanol, Isopropanol, n-Butanol und tert.-Butanol.

Die Reaktionstemperaturen können bei der Durchführung der ersten Stufe des erfindungsgemäßen Verfahrens innerhalb eines größeren Bereiches variiert werden. Im allgemeinen arbeitet man bei Temperaturen zwischen 0 ° C und 100 ° C, vorzugsweise zwischen 10 ° C und 60 ° C.

Bei der Durchführung der ersten Stufe des erfindungsgemäßen Verfahrens arbeitet man im allgemeinen unter Normaldruck. Es ist jedoch auch möglich, unter erhöhtem oder vermindertem Druck zu arbeiten.

Bei der Durchführung der ersten Stufe des erfindungsgemäßen Verfahrens geht man im allgemeinen so vor, daß man auf 1 Mol an Phenol-Derivat 1 Mol an Base einsetzt. Es ist aber auch möglich, die eine oder andere Komponente in einem Überschuß einzusetzen. Die Aufarbeitung erfolgt nach üblichen Methoden. Im allgemeinen geht man so vor, daß man das Reaktionsgemisch durch Abziehen des Verdünnungsmittels einengt und das dabei verbleibende Phenolat entweder direkt oder nach vorheriger Reinigung für die weitere Synthese verwendet.

Die Halogenverbindungen, die bei der Durchführung der zweiten Stufe des erfindungsgemäßen Verfahrens als Reaktionskomponenten dienen, sind durch die Formel (III) allgemein definiert. In dieser Formel haben $R^1$, $R^2$ und $R^3$ vorzugsweise diejenigen Bedeutungen, die bereits im Zusammenhang mit der Beschreibung der erfindungsgemäßen Stoffe der Formel (I) vorzugsweise für diese Reste genannt wurden. Hal steht für Chlor, Brom oder lod.

Die Halogenverbindungen der Formel (III) sind bekannt oder lassen sich nach prinzipiell bekannten Verfahren herstellen.

Als Verdünnungsmittel kommen bei der Durchführung der zweiten Stufe des erfindungsgemäßen Verfahrens alle für derartige Umsetzungen üblichen organischen Solventien in Betracht. Vorzugsweise verwendbar sind Alkohole, wie Methanol, Ethanol und Butanol, weiterhin Ether, wie Diethylester, Dioxan oder Tetrahydrofuran, ferner halogenierte aliphatische Kohlenwasserstoffe, wie Methylenchlorid, Chloroform oder Tetrachlorkohlenstoff, außerdem Nitrile, wie Acetonitril oder Propionitril, darüber hinaus Amide, wie Dimethylformamid, sowie stark polare Solventien, wie Dimethylsulfoxid oder Hexamethylphosphorsäure-triamid.

Als Säurebindemittel kommen bei der Durchführung der zweiten Stufe des erfindungsgemäßen Verfahrens alle üblichen Säureakzeptoren in Betracht. Vorzugsweise verwendbar sind Alkalimetallcarbonate und Hydrogencarbonate, wie Natriumcarbonat, Kaliumcarbonat oder Natriumhydrogencarbonat, ferner Alkalimetallhydroxide und -Alkoholate, wie Natriumhydroxid, Kaliumhydroxid, Natriummethylat oder Kalium-tert.-butylat, außerdem tertiäre aliphatische oder aromatische Amine, wie Triethylamin, N,N-Dimethyl-cyclohexylamin, N,N-Dimethyl-benzylamin und Pyridin, und außerdem cyclische Amine, wie 1,5-Diaza-bicyclo[4.3.0]-non-5-en (DBN), 1,8-Diaza-bicyclo[5.4.0]undec-7-en (DBU) und 1,4-Diaza-bicyclo[2.2.2]octan (DABCO).

Die Reaktionstemperaturen können bei der Durchführung der zweiten Stufe des erfindungsgemäßen Verfahrens innerhalb eines größeren Bereiches variiert werden. Im allgemeinen arbeitet man bei Temperaturen zwischen 0 ° C und 120 ° C, vorzugsweise zwischen 10 ° C und 100 ° C.

Bei der Durchführung der zweiten Stufe des erfindungsgemäßen Verfahrens arbeitet man im allgemeinen unter Normaldruck. Es ist aber auch möglich, unter erhöhtem oder vermindertem Druck zu arbeiten.

Bei der Durchführung der zweiten Stufe des erfindungsgemäßen Verfahrens setzt man auf 1 Mol an Phenolat der Formel (II-a) im allgemeinen 1 bis 1,3 Mol an Halogenverbindung der Formel (III) ein. Die Aufarbeitung erfolgt nach üblichen Methoden. Im allgemeinen geht man so vor, daß man das Reaktionsgemisch durch Abziehen des Verdünnungsmittels einengt und den verbleibenden Rückstand durch Umkristallisation oder auf chromatographischem Wege reinigt.

Die nach dem erfindungsgemäßen Verfahren erhältlichen Verbindungen der Formel (I) können in Säureadditions-Salze bzw. Metallsalz-Komplexe überführt werden.

Zur Herstellung von Säureadditions-Salzen der Verbindungen der Formel (I) kommen vorzugsweise diejenigen Säuren in Frage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z.B. durch Lösen einer Verbindung der allgemeinen Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der allgemeinen Formel (I) kommen vorzugsweise diejenigen Salze von Metallen in Frage, die bereits weiter oben beschrieben wurden.

Die Metallsalz-Komplexe von Verbindungen der allgemeinen Formel (I) können in einfacher Weise nach üblichen Verfahren erhalten werden, so z.B. durch Lösen des Metallsalzes in Alkohol, z.B. Ethanol, und Hinzufügen zu Verbindungen der allgemeinen Formel (I). Man kann Metallsalz-Komplexe in bekannter Weise, z.B. durch Abfiltrieren, isolieren und gegebenenfalls durch Umkristallisation reinigen.

Die erfindungsgemäßen Wirkstoffe wiesen eine starke mikrobizide Wirkung auf und können als Fungizide im Pflanzenschutz und im Materialschutz eingesetzt werden.

Fungizide werden im Pflanzenschutz eingesetzt zur Bekämpfung von Plasmodiophoromycetes, Oomycetes, Chytridiomycetes, Zygomycetes, Ascomycetes, Basidiomycetes, Deuteromycetes.

Beispielhaft seien einige Erreger von pilzlichen und bakteriellen Erkrankungen, die unter die oben aufgezählten Oberbegriffe fallen, genannt:
Xanthomonas-Arten, wie Xanthomonas oryzae;
Pseudomonas-Arten, wie Pseudomonas lachrymans;
Erwinia-Arten, wie Erwinia amylovora;
Pythium-Arten, wie Pythium ultimum;
Phytophthora-Arten, wie Phytophthora infestans;
Pseudoperonospora-Arten, wie Pseudoperonospora humuli oder Pseudoperonospora cubense;
Plasmopara-Arten, wie Plasmopara viticola;
Peronospora-Arten, wie Peronospora pisi oder P. brassicae;
Erysiphe-Arten, wie Erysiphe graminis;
Sphaerotheca-Arten, wie Sphaerotheca fuliginea;
Podosphaera-Arten, wie Podosphaera leucotricha;
Venturia-Arten, wie Venturia inaequalis;
Pyrenophora-Arten, wie Pyrenophora teres oder P. graminea;
(Konidienform: Drechslera, Syn: Helminthosporium);
Cochliobolus-Arten, wie Cochliobolus sativus;
(Konidienform: Drechslera, Syn: Helminthosporium);
Uromyces-Arten, wie Uromyces appendiculatus;
Puccinia-Arten, wie Puccinia recondita;
Tilletia-Arten, wie Tilletia caries;
Ustilago-Arten, wie Ustilago nuda oder Ustilago avenae;
Pellicularia-Arten, wie Pellicularia sasakii;
Pyricularia-Arten, wie Pyricularia oryzae;
Fusarium-Arten, wie Fusarium culmorum;
Botrytis-Arten, wie Botrytis cinerea;
Septoria-Arten, wie Septoria nodorum;
Leptosphaeria-Arten, wie Leptosphaeria nodorum;
Cercospora-Arten, wie Cercospora canescens;
Alternaria-Arten, wie Alternaria brassicae;
Pseudocercosporella-Arten, wie Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Die erfindungsgemäßen Stoffe zeigen im Pflanzenschutz eine besonders gute Wirkung gegen Fusarium-Pilze sowie gegen Pyricularia und Botrytis. Außerdem besitzen sie starke bakterizide Eigenschaften und erweisen sich im Agarplattentest als sehr wirksam.

Im Materialschutz lassen sich die erfindungsgemäßen Wirkstoffe zum Schutz von technischen Materialien einsetzen. Unter technischen Materialien sind in diesem Zusammenhang nicht lebende Materialien zu verstehen, die für die Verwendung in der Technik zubereitet worden sind. Beispielsweise können technische Materialien, die durch die erfindungsgemäßen Wirkstoffe vor mikrobieller Veränderung oder Zerstörung geschützt werden sollen, Klebstoffe, Leime, Papier, Karton, Textilien, Leder, Holz, Anstrichmittel, Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Kühlkreisläufe genannt, besonders bevorzugt Holz.

Als Mirkoorganismen, die einem Abbau oder eine Veränderung der technischen Materialien bewirken können, seien beispielsweise Bakterien, Pilze, Hefen, Algen und Schleimorganismen genannt. Vorzugsweise

wirken die erfindungsgemäßen Wirkstoffe gegen Pilze, insbesondere Schimmelpilze, holzverfärbende und holzzerstörende Pilze (Basidiomyceten), sowie gegen Schleimorganismen und Algen.

Es seien beispielsweise Mikroorganismen der folgenden Gattungen genannt:
Alternaria, wie Alternaria tenuis,
Aspergillus, wie Aspergillus niger,
Chaetomium, wie Chaetomium globosum,
Coniophora, wie Coniophora puteana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Die erfindungemäßen Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie Mischungen mit Düngemitteln und anderen Wachstumsregulatoren.

Die Wirkstoffe können als solche, in Form ihrer Formulierungen oder den daraus bereiteten Anwendungsformen wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Stoffe als Fungizide kann die Aufwandmenge je nach Art der Applikation in einem größeren Bereich variiert werden. So liegen die Wirkstoffkonzentrationen bei der Behandlung von Pflanzenteilen in den Anwendungsformen im allgemeinen zwischen 1 und 0,0001 Gew.-%, vorzugsweise zwischen 0,5 und 0,001 %. Bei der Saatgutbehandlung werden im allgemeinen Wirkstoffmengen von 0,001 bis 50 g je Kilogramm Saatgut, vorzugsweise 0,01 bis 10 g, benötigt. Bei Behandlung des Bodens sind Wirkstoffkonzentrationen von 0,00001 bis 0,1 Gew.-%, vorzugsweise von0,0001 bis 0,02 %, am Wirkungsort erforderlich.

Die Herstellung und die Verwendung der erfindungsgemäßen Wirkstoffe wird durch die folgenden Beispiele veranschaulicht.

Herstellungsbeispiele

Referenzbeispiel 1

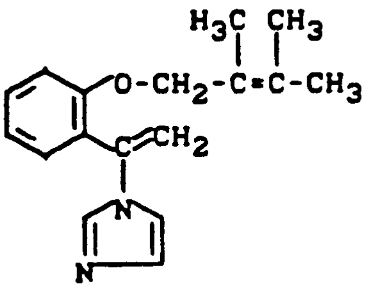

(1)

In ein Gemisch aus 39,1 g (0,21 Mol) 1-[1-(2-Hydroxyphenyl)-vinyl]-imidazol und 200 ml Methanol werden bei Raumtemperatur unter Rühren 9,2 g (0,23 Mol) Natriumhydroxid gegeben. Nach beendeter Zugabe wird noch 30 Minuten bei 50 °C gerührt und dann durch Abziehen des Lösungsmittels unter vermindertem Druck eingeengt. Der verbleibende feste Rückstand wird in 200 ml Dimethylformamid aufgenommen. In die entstehende Lösung werden unter Rühren bei einer Temperatur von 25 °C 24,9 g (0,21 Mol) 1-Chlor-2,3-dimethyl-but-2-en gegeben. Es wird noch 12 Stunden bei 20 °C gerührt und dann aufgearbeitet, indem man das Reaktionsgemisch durch Abziehen des Verdünnungsmittels einengt. Der anfallende Rückstand wird über Kieselgel chromatographiert. Man erhält auf diese Weise 35,5 g (63 % der Theorie) an 1-[1-(2-(2,3-Dimethyl-but-2-en-1-yl-oxy)-phenyl)-vinyl]-imidazol in Form eines Öles.

$^1$H-NMR (DMSO):

$\delta$ = 4,38 ppm (s) [O-$CH_2$-C = C]

Nach der im Referenzbeispiel 1 angegebenen Methode werden auch die in der folgenden Tabelle 1 aufgeführten Stoffe der Formel

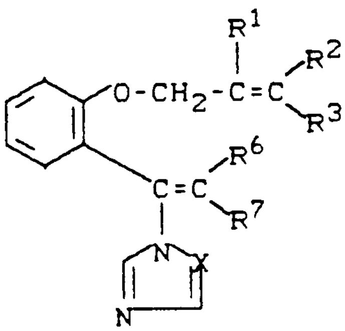

(I-a)

erhalten.

**Tabelle 1 (Fortsetzung)**

| Bsp. | $R^1$ | $R^2$ | $R^3$ | $R^6$ | $R^7$ | X | $^1H$-NMR (DMSO) δ ($O-CH_2-C=C$) |
|---|---|---|---|---|---|---|---|
| 31 | H | H | H | $C_6H_5$ | H | CH | 4,52 (d) |
| 32 | $CH_3$ | H | H | $C_6H_5$ | H | CH | 4,46 (s) |
| 33 | H | $CH_3$ | $CH_3$ | $C_6H_5$ | H | CH | 4,49 (d) |
| 34 | $CH_3$ | $CH_3$ | $CH_3$ | $C_6H_5$ | H | CH | 4,50 (s) |
| 35 | Cl | H | H | $C_6H_5$ | H | CH | 4,72 (s) |
| 36 | Br | H | H | $C_6H_5$ | H | CH | 4,77 (s) |

Nach der im Referenzbeispiel 1 angegebenen Methode werden auch die in der folgenden Tabelle 3 aufgeführten Stoffe der Formel

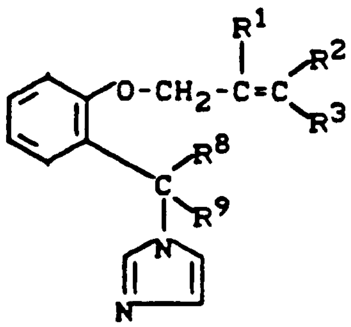

(I-c)

erhalten.

Tabelle 3

| Bsp. Nr. | $R^1$ | $R^2$ | $R^3$ | $R^8$ | $R^9$ | $^1$H-NMR (DMSO) δ ($O-CH_2-C=C$) |
|---|---|---|---|---|---|---|
| 44 | H | H | H | $CH_3$ | H | 4,59 (d) |
| 45 | $CH_3$ | H | H | $CH_3$ | H | 4,50 (s) |
| 46 | Cl | H | H | $CH_3$ | H | 4,75 (s) |
| 47 | H | $CH_3$ | $CH_3$ | $CH_3$ | H | 4,55 (d) |
| 48 | $CH_3$ | $CH_3$ | $CH_3$ | $CH_3$ | H | 4,52 (s) |
| 49 | Cl | Cl | Cl | $CH_3$ | H | 5,02 (s) |

Nach der im Referenzbeispiel 1 angegebenen Methode werden auch die in der folgenden Tabelle 4 aufgeführten Stoffe der Formel

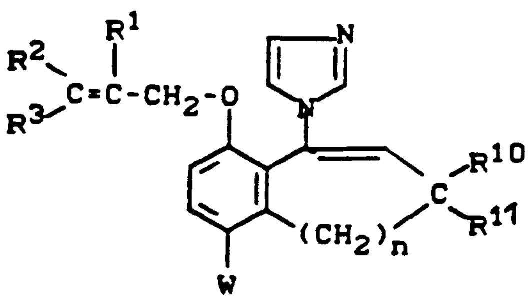

(I-d)

erhalten.

Tabelle 4

| Bsp. Nr. | $R^1$ | $R^2$ | $R^3$ | $R^{10}$ | $R^{11}$ | n | W | $^1H$-NMR (DMSO) δ ($O-CH_2-C\equiv C$) |
|---|---|---|---|---|---|---|---|---|
| 50 | H | H | H | H | H | 1 | H | 4,25 (d) |
| 51 | H | $CH_3$ | $CH_3$ | H | H | 1 | H | 4,19 (d) |
| 52 | Cl | H | H | H | H | 1 | H | 4,35 (s) |
| 53 | H | $CH_3$ | $CH_3$ | $CH_3$ | $CH_3$ | 0 | Cl | 4,43 (d) |
| 54 | Cl | H | H | $CH_3$ | $CH_3$ | 0 | Cl | 4,60 (s) |

Herstellung von Ausgangssubstanzen:

Beispiel 55

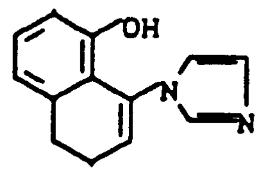

In ein Gemisch aus 503 g (7,4 Mol) Imidazol und 1,6 Litern Methylenchlorid werden bei 5 ° C unter Rühren 227 g (1,9 Mol) Thionylchlorid eingetropft. Man rührt 20 Minuten nach und gibt dann 157 g (0,97 Mol) 8-Hydroxy-1-tetralon hinzu. Das Reaktionsgemisch wird noch 2 Stunden gerührt, dann filtriert und in Eiswasser gegossen. Die organische Phase wird abgetrennt und unter vermindertem Druck eingeengt. Man nimmt den Rückstand in 1 Liter Toluol auf und kocht die entstehende Lösung eine Stunde unter Rückfluß. Das Gemisch wird erneut unter vermindertem Druck eingeengt, und der verbleibende Rückstand wird in Methylenchlorid gelöst. Die organische Phase wird mit Wasser gewaschen und unter vermindertem Druck eingeengt. Der Rückstand wird mit Toluol verrieben, wobei sich das Produkt kristallin abscheidet. Man erhält auf diese Weise 95 g 3,4-Dihydro-1-(imidazol-1-yl)-8-hydroxy-naphthalin vom Schmelzpunkt 186 ° C.

Beispiel 56

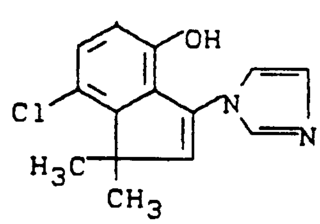

Nach der im Beispiel 55 angegebenen Methode wird aus 3,3-Dimethyl-4-chlor-7-hydroxy-indanon das 1-(Imidazol-1 yl)-3,3-dimethyl-4-chlor-7-hydroxy-inden in Form farbloser Kristalle hergestellt.

In den folgenden Verwendungsbeispielen wurden die nachstehend angegebenen Verbindungen als Vergleichssubstanzen eingesetzt.

(A) =

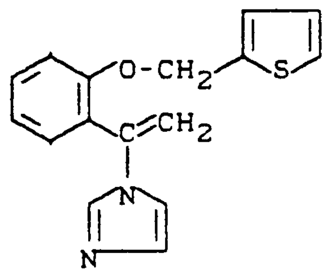

(B) =

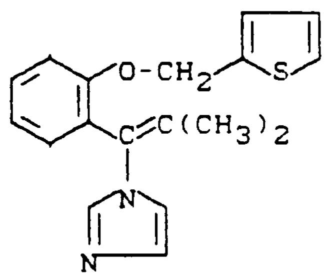

(C) =

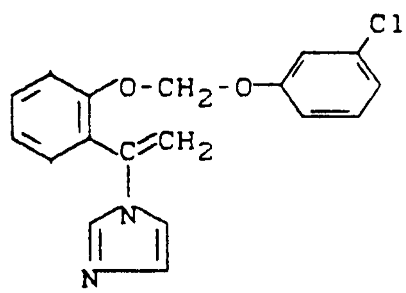

Beispiel B

Pyricularia-Test (Reis) /protektiv

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25 ° C aufgestellt.

4 Tage nach der Inokulation erfolgt die Auswertung des Krankheitsbefalls.

In diesem Test zeigen die erfindungsgemäßen Substanzen 46 und 49 eine wesentlich bessere Wirkung als die Vergleichssubstanz C.

**Patentansprüche**
**Patentansprüche für folgende Vertragsstaaten : BE, CH, DE, FR, GB, GR, IT, LI, NL**

**1.** (Azolyl-vinyl)-phenol-alkenylether der Formel

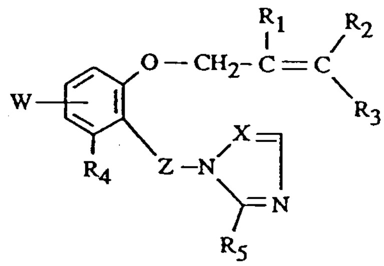

(I)

in welcher

- $R_1$ für Wasserstoff, Fluor, Chlor, Brom oder $C_1$-$C_4$-Alkyl steht,
- $R_2$ für Wasserstoff, Fluor, Chlor, Brom, $C_1$-$C_4$-Alkyl, $C_2$-$C_4$-Alkenyl, wobei jeder dieser Alkenylreste substituiert sein kann durch gegebenenfalls substituiertes Aryl, oder für gegebenenfalls einfach bis dreifach, gleichartig oder verschieden durch $C_1$-$C_4$-Alkyl, Halogen und/oder Halogen-$C_1$-$C_2$-alkyl mit 1 bis 5 Halogenatomen substituiertes Phenyl steht, oder
- $R^1$ und $R_2$ gemeinsam für eine C-C-Bindung stehen,
- $R_3$ für Wasserstoff Fluor, Chlor, Brom, Iod, $C_1$-$C_{12}$-Alkyl oder für gegebenenfalls einfach bis dreifach gleichartig oder verschieden durch $C_1$-$C_4$-Alkyl, Halogen und/oder Halogen-$C_1$-$C_2$-alkyl mit 1 bis 5 Halogenatomen substituiertes Phenyl steht,
- $R_4$ für Wasserstoff steht,
- $R_5$ für Wasserstoff oder $C_1$-$C_4$-Alkyl steht,
- W für Wasserstoff, Fluor, Chlor oder Brom steht,
- X für Stickstoff oder eine CH-Gruppe steht und
- Z für die Gruppierungen

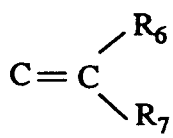 oder 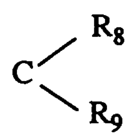

steht, worin

- $R_6$ für gegebenenfalls einfach bis dreifach, gleichartig oder verschieden durch $C_1$-$C_4$-Alkyl, Halogen und/oder Halogen-$C_1$-$C_2$-alkyl mit 1 bis 5 Halogenatomen substituiertes Phenyl steht oder
- $R_6$ gemeinsam mit $R_4$ für die Gruppierung

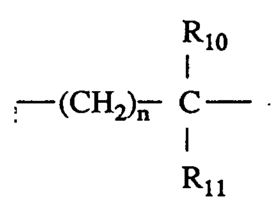

steht,

- $R_7$ für Wasserstoff oder $C_1$-$C_4$-Alkyl steht,
- $R_8$ für Wasserstoff oder $C_1$-$C_4$-Alkyl steht,
- $R_9$ für Wasserstoff oder $C_1$-$C_4$-Alkyl steht,
- $R_{10}$ für Wasserstoff oder $C_1$-$C_4$-Alkyl steht,
- $R_{11}$ für Wasserstoff oder $C_1$-$C_4$-Alkyl steht,
- n für Null oder die Zahlen 1 oder 2 steht,

sowie deren Säureadditions-Salze und Metallsalz-Komplexe.

**2.** (Azolyl-vinyl)-phenol-alkenylether der Formel (I) gemäß Anspruch 1, in denen

$R_1$ für Wasserstoff, Fluor, Chlor, Brom, Methyl, Ethyl, n-Propyl, Isopropyl oder n-Butyl steht,

$R_2$ für Wasserstoff, Fluor, Chlor, Brom, Methyl, Ethyl, n-Propyl, Isopropyl, n-Butyl, Vinyl, Propenyl oder Styryl steht, oder für gegebenenfalls einfach bis dreifach, gleichartig oder verschieden durch Methyl, Ethyl, Fluor, Chlor, Brom und/oder Trifluormethyl substituiertes Phenyl steht oder

$R_1$ und $R_2$ gemeinsam für eine C-C-Bindung,

$R_3$ für Wasserstoff, Fluor, Chlor, Brom, Iod, $C_1$-$C_{10}$-Alkyl oder für gegebenenfalls einfach bis dreifach, gleichartig oder verschieden durch Methyl, Ethyl, Fluor, Chlor, Brom und/oder Trifluormethyl substituiertes Phenyl steht,

$R_4$ für Wasserstoff steht,

$R_5$ für Wasserstoff, Methyl oder Ethyl steht,

W für Wasserstoff oder Chlor steht,

X für Stickstoff oder eine CH-Gruppe steht und

Z für die Gruppierungen

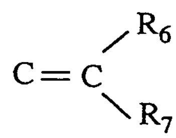

oder

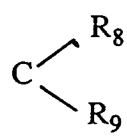

steht, worin

$R_6$ für gegebenenfalls einfach bis dreifach, gleichartig oder verschieden durch Methyl, Ethyl, Fluor, Chlor und/oder Trifluormethyl substituiertes Phenyl steht oder

$R_6$ gemeinsam mit $R_4$ für die Gruppierung

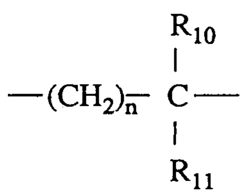

steht,

$R_7$ für Wasserstoff, Methyl, Ethyl, n-Propyl, Isopropyl oder n-Butyl steht,

$R_8$ für Wasserstoff, Methyl, Ethyl, n-Propyl, Isopropyl oder n-Butyl steht,

$R_9$ für Wasserstoff, Methyl, Ethyl, n-Propyl, Isopropyl oder n-Butyl steht,

$R_{10}$ für Wasserstoff oder Methyl steht,

$R_{11}$ für Wasserstoff oder Methyl steht und

n für Null oder die Zahl 1 steht,

**3.** Verfahren zur Herstellung von (Azolyl-vinyl)-phenol-alkylenethern nach Anspruch 1, sowie von deren Säureadditions-Salzen und Metallsalz-Komplexen, dadurch gekennzeichnet, daß man Phenolderivate der Formel

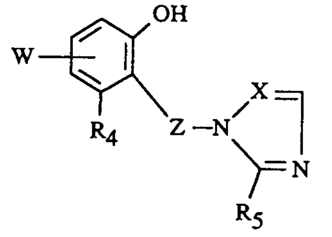

(II)

in welcher

$R_4$, $R_5$, W, X und Z die oben angegebene Bedeutung haben,
mit Basen gegebenenfalls in Gegenwart eines Verdünnungsmittels umsetzt und die dabei entstehenden Phenolate der Formel

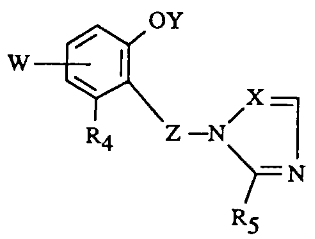

(IIa)

in welcher
$R_4$, $R_5$, W, X und Z die oben angegebene Bedeutung haben und
Y für einen Baserest steht,
mit Halogenverbindungen der Formel

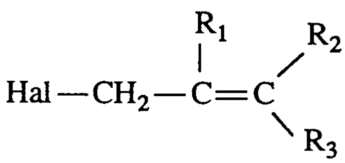

(III)

in welcher
$R_1$, $R_2$ und $R_3$ die oben angegebene Bedeutung haben und
Hal für Chlor, Brom oder Iod steht,
in Gegenwart eines Verdünnungsmittels sowie gegebenenfalls in Gegenwart eines Säurebindemittels umsetzt und gegebenenfalls anschließend an die so erhaltenen Verbindungen der Formel (I) eine Säure oder ein Metallsalz addiert.

**4.** Mikrobizide Mittel, gekennzeichnet durch einen Gehalt an mindestens einem (Azolyl-vinyl)-phenol-alkenylether der Formel (I) gemäß Anspruch 1 bzw. an einem Säureadditions-Salz oder Metallsalz-Komplex eines (Azoylvinyl)-phenol-alkenylethers der Formel (I).

**5.** Verwendung von (Azolyl-vinyl)-phenol-alkenylethern der Formel (I) gemäß Anspruch 1 bzw. von deren Säureadditions-Salzen und Metall-Komplexen als Mikrobizide im Pflanzenschutz und im Materialschutz.

**6.** Verfahren zur Bekämpfung von unerwünschten Mikroorganismen im Pflanzenschutz und im Materialschutz, dadurch gekennzeichnet, daß man (Azolyl-vinyl)-phenol-alkenylether der Formel (I) gemäß Anspruch 1 bzw. deren Säureadditions-Salze oder Metallsalz-Komplexe auf die Mikroorganismen und/oder deren Lebensraum ausbringt.

**7.** Verfahren zur Herstellung von mikrobiziden Mitteln, dadurch gekennzeichnet, daß man (Azolyl-vinyl)-phenol-alkenylether der Formel (I) gemäß Anspruch 1 bzw. deren Säureadditions-Salze oder Metallsalz-Komplexe mit Streckmitteln und/oder obenflächenaktiven Stoffen vermischt.

**8.** (Azolyl-vinyl)-phenol-alkenylether gemäß Anspruch 1, gekennzeichnet durch die Formel

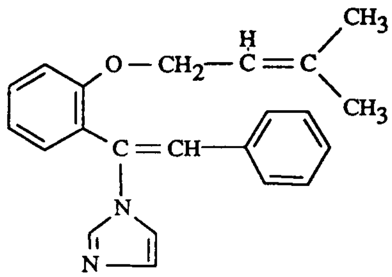

9. (Azolyl-vinyl)-phenol-alkenylether gemäß Anspruch 1, gekennzeichnet durch die Formel

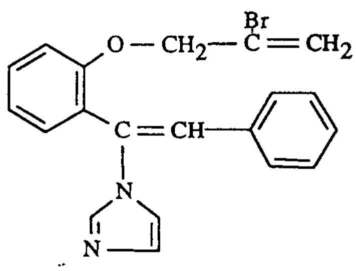

**Patentansprüche für folgenden Vertragsstaat : ES**

1. Verfahren zur Herstellung von (Azolyl-vinyl)-phenol-alkenylether der Formel

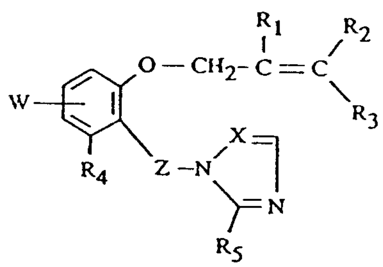

(I)

in welcher

| | |
|---|---|
| $R_1$ | für Wasserstoff, Fluor, Chlor, Brom oder $C_1$-$C_4$-Alkyl steht, |
| $R_2$ | für Wasserstoff, Fluor, Chlor, Brom, $C_1$-$C_4$-Alkyl, $C_2$-$C_4$-Alkenyl, wobei jeder dieser Alkenylreste substituiert sein kann durch gegebenenfalls substituiertes Aryl, oder für gegebenenfalls einfach bis dreifach, gleichartig oder verschieden durch $C_1$-$C_4$-Alkyl, Halogen und/oder Halogen-$C_1$-$C_2$-alkyl mit 1 bis 5 Halogenatomen substituiertes Phenyl steht, oder |
| $R_1$ und $R_2$ | gemeinsam für eine C-C-Bindung stehen, |
| $R_3$ | für Wasserstoff, Fluor, Chlor, Brom, Iod, $C_1$-$C_{12}$-Alkyl oder für gegebenenfalls einfach bis dreifach gleichartig oder verschieden durch $C_1$-$C_4$-Alkyl, Halogen und/oder Halogen-$C_1$-$C_2$-alkyl mit 1 bis 5 Halogenatomen substituiertes Phenyl steht, |
| $R_4$ | für Wasserstoff steht, |
| $R_5$ | für Wasserstoff oder $C_1$-$C_4$-Alkyl steht, |
| W | für Wasserstoff, Fluor, Chlor oder Brom steht, |
| X | für Stickstoff oder eine CH-Gruppe steht und |
| Z | für die Gruppierungen |

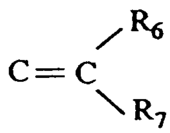 oder 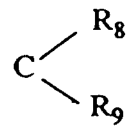

steht, worin

$R_6$ für gegebenenfalls einfach bis dreifach, gleichartig oder verschieden durch $C_1$-$C_4$-Alkyl, Halogen und/oder Halogen-$C_1$-$C_2$-alkyl mit 1 bis 5 Halogenatomen substituiertes Phenyl steht oder

$R_6$ gemeinsam mit $R_4$ für die Gruppierung

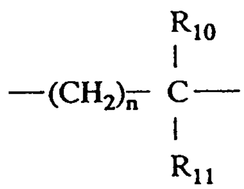

steht,

$R_7$ für Wasserstoff oder $C_1$-$C_4$-Alkyl steht,

$R_8$ für Wasserstoff oder $C_1$-$C_4$-Alkyl steht,

$R_9$ für Wasserstoff oder $C_1$-$C_4$-Alkyl steht,

$R_{10}$ für Wasserstoff oder $C_1$-$C_4$-Alkyl steht,

$R_{11}$ für Wasserstoff oder $C_1$-$C_4$-Alkyl steht,

n für Null oder die Zahlen 1 oder 2 steht,

sowie von deren Säureadditions-Salzen und Metallsalz-Komplexen, dadurch gekennzeichnet, daß man Phenolderivate der Formel

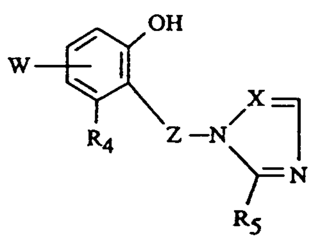

(II)

in welcher

$R_4$, $R_5$, W, X und Z die oben angegebene Bedeutung haben,

mit Basen gegebenenfalls in Gegenwart eines Verdünnungsmittels umsetzt und die dabei entstehenden Phenolate der Formel

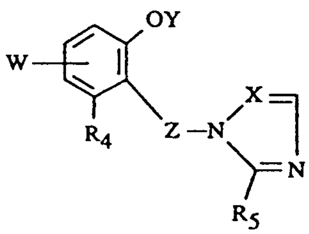

(IIa)

in welcher

$R_4$, $R_5$, W, X und Z die oben angegebene Bedeutung haben und

Y für einen Baserest steht,
mit Halogenverbindungen der Formel

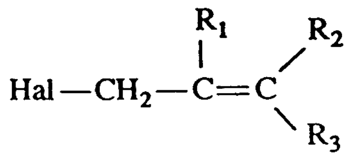

(III)

in welcher
$R_1$, $R_2$ und $R_3$ die oben angegebene Bedeutung haben und
Hal für Chlor, Brom oder lod steht,
in Gegenwart eines Verdünnungsmittels sowie gegebenenfalls in Gegenwart eines Säurebindemittels umsetzt und gegebenenfalls anschließend an die so erhaltenen Verbindungen der Formel (I) eine Säure oder ein Metallsalz addiert.

2. Mikrobizide Mittel, gekennzeichnet durch einen Gehaltan mindestens einem (Azolyl-vinyl)-phenol-alkenylether der Formel (I) gemäß Anspruch 1 bzw. an einem Säureadditions-Salz oder Metallsalz-Komplex eines (Azoylvinyl)-phenol-alkenylethers der Formel (I).

3. Verwendung von (Azolyl-vinyl)-phenol-alkenylethern der Formel (I) gemäß Anspruch 1 bzw. von deren Säureadditions-Salzen und Metall-Komplexen als Mikrobizide im Pflanzenschutz und im Materialschutz.

4. Verfahren zur Bekämpfung von unerwünschten Mikroorganismen im Pflanzenschutz und im Materialschutz, dadurch gekennzeichnet, daß man (Azolyl-vinyl)phenol-alkenylether der Formel (I) gemäß Anspruch 1 bzw. deren Säureadditions-Salze oder Metallsalz-Komplexe auf die Mikroorganismen und/oder deren Lebensraum ausbringt.

5. Verfahren zur Herstellung von mikrobiziden Mitteln, dadurch gekennzeichnet, daß man (Azolyl-vinyl)-phenol-alkenylether der Formel (I) gemäß Anspruch 1 bzw. deren Säureadditions-Salze oder Metallsalz-Komplexe mit Streckmitteln und/oder obenflächenaktiven Stoffen vermischt.

## Claims

**Claims for the following Contracting States : BE, CH, DE, FR, GB, GR, IT, LI, NL**

1. (Azolyl-vinyl)-phenol alkenyl ethers of the formula

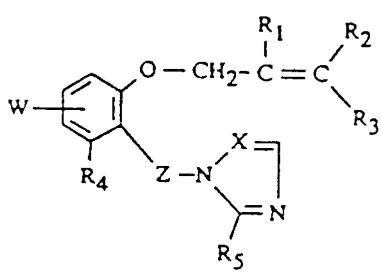

(I)

in which

| | |
|---|---|
| $R_1$ | represents hydrogen, fluorine, chlorine, bromine or $C_1$-$C_4$-alkyl, |
| $R_2$ | represents hydrogen, fluorine, chlorine, bromine, $C_1$-$C_4$-alkyl or $C_2$-$C_4$-alkenyl, it being possible for each of these alkenyl radicals to be substituted by optionally substituted aryl, or represents phenyl which is optionally substituted by one to three identical or different substituents from the group comprising $C_1$-$C_4$-alkyl, halogen and/or halogeno-$C_1$-$C_2$-alkyl with 1 to 5 halogen atoms, or |
| $R_1$ and $R_2$ | together represent a C-C bond, |

$R_3$ represents hydrogen, fluorine, chlorine, bromine, iodine or $C_1$-$C_{12}$-alkyl, or represents phenyl which is optionally substituted by one to three identical or different substituents from the group comprising $C_1$-$C_4$-alkyl, halogen and/or halogeno-$C_1$-$C_2$-alkyl with 1 to 5 halogen atoms,

$R_4$ represents hydrogen,

$R_5$ represents hydrogen or $C_1$-$C_4$-alkyl,

W represents hydrogen, fluorine, chlorine or bromine,

X represents nitrogen or a CH group and

Z represents the groupings

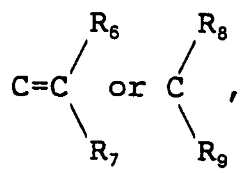

wherein

$R_6$ represents phenyl which is optionally substituted by one to three identical or different substituents from the group comprising $C_1$-$C_4$-alkyl, halogen and/or halogeno-$C_1$-$C_2$-alkyl with 1 to 5 halogen atoms, or

$R_6$ together with $R_4$ represents the grouping

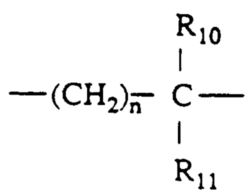

$R_7$ represents hydrogen or $C_1$-$C_4$-alkyl,

$R_8$ represents hydrogen or $C_1$-$C_4$-alkyl,

$R_9$ represents hydrogen or $C_1$-$C_4$-alkyl,

$R_{10}$ represents hydrogen or $C_1$-$C_4$-alkyl,

$R_{11}$ represents hydrogen or $C_1$-$C_4$-alkyl and

n represents zero or the numbers 1 or 2,

and acid addition salts and metal salt complexes thereof.

**2.** (Azolyl-vinyl)-phenol alkenyl ethers of the formula (I) according to Claim 1, in which

$R_1$ represents hydrogen, fluorine, chlorine, bromine, methyl, ethyl, n-propyl, isopropyl or n-butyl,

$R_2$ represents hydrogen, fluorine, chlorine, bromine, methyl, ethyl, n-propyl, isopropyl, n-butyl, vinyl, propenyl or styryl, or represents phenyl which is optionally substituted by one to three identical or different substituents from the group comprising methyl, ethyl, fluorine, chlorine, bromine and/or trifluoromethyl, or

$R_1$ and $R_2$ together represent a C-C bond,

$R_3$ represents hydrogen, fluorine, chlorine, bromine, iodine or $C_1$-$C_{10}$-alkyl, or represents phenyl which is optionally substituted by one to three identical or different substituents from the group comprising methyl, ethyl, fluorine, chlorine, bromine and/or trifluoromethyl,

$R_4$ represents hydrogen,

$R_5$ represents hydrogen, methyl or ethyl,

W represents hydrogen or chlorine,

X represents nitrogen or a CH group and

Z represents the groupings

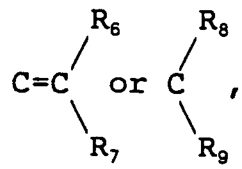

wherein

$R_6$ represents phenyl which is optionally substituted by one to three identical or different substituents from the group comprising methyl, ethyl, fluorine, chlorine and/or trifluoromethyl, or

$R_6$ together with $R_4$ represents the grouping

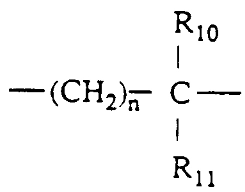

$R_7$ represents hydrogen, methyl, ethyl, n-propyl, isopropyl or n-butyl,

$R_8$ represents hydrogen, methyl, ethyl, n-propyl, isopropyl or n-butyl,

$R_9$ represents hydrogen, methyl, ethyl, n-propyl, isopropyl or n-butyl,

$R_{10}$ represents hydrogen or methyl,

$R_{11}$ represents hydrogen or methyl and

n represents zero or the number 1.

3. Process for the preparation of (azolyl-vinyl)-phenol alkenyl ethers according to Claim 1, and of acid addition salts and metal salt complexes thereof, characterised in that phenol derivatives of the formula

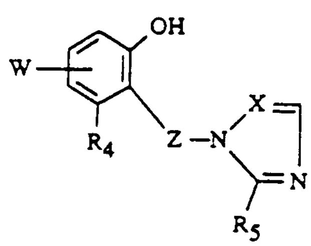

(II)

in which

$R_4$, $R_5$, W, X and Z have the abovementioned meaning,

are reacted with bases, if appropriate in the presence of a diluent, and the phenolates thereby formed, of the formula

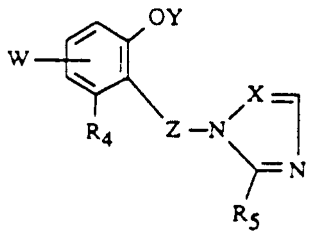

(IIa)

in which

$R_4$, $R_5$, W, X and Z have the abovementioned meaning and

Y represents a base radical,

are reacted with halogen compounds of the formula

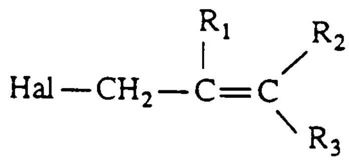

(III)

in which

$R_1$, $R_2$ and $R_3$ have the abovementioned meaning and

Hal represents chlorine, bromine or iodine,

in the presence of a diluent and if appropriate in the presence of an acid-binding agent, and, if appropriate, an acid or a metal salt is then added onto the compounds of the formula (I) thus obtained.

4. Microbicidal agents, characterised in that they contain at least one (azolyl-vinyl)-phenol alkenyl ether of the formula (I) according to Claim 1 or an acid addition salt or metal salt complex of an (azolyl-vinyl)-phenol alkenyl ether of the formula (I).

5. Use of (azolyl-vinyl)-phenol alkenyl ethers of the formula (I) according to Claim 1 or of acid addition salts and metal complexes thereof as microbicides in plant protection and the preservation of materials.

6. Method of combating undesirable microorganisms in plant protection and the preservation of materials, characterised in that (azolyl-vinyl)-phenol alkenyl ethers of the formula (I) according to Claim 1 or acid addition salts or metal salt complexes thereof are applied to the microorganisms and/or their environment.

7. Process for the preparation of microbicidal agents, characterised in that (azolyl-vinyl)-phenol alkenyl ethers of the formula (I) according to Claim 1 or acid addition salts or metal salt complexes thereof are mixed with extenders and/or surface-active substances.

8. (Azolyl-vinyl)-phenol alkenyl ethers according to Claim 1, characterised by the formula

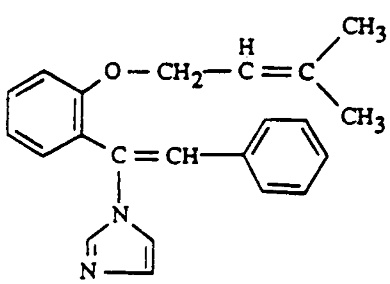

9. (Azolyl-vinyl)-phenol alkenyl ethers according to Claim 1, characterised by the formula

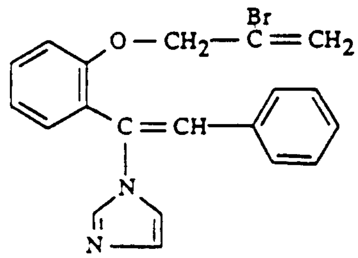

**Claims for the following Contracting State : ES**

1. Process for the preparation of (azolyl-vinyl)-phenol alkenyl ether of the formula

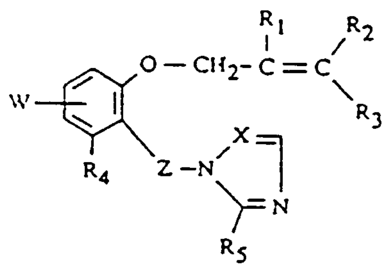

(I)

in which

| | |
|---|---|
| $R_1$ | represents hydrogen, fluorine, chlorine, bromine or $C_1$-$C_4$-alkyl, |
| $R_2$ | represents hydrogen, fluorine, chlorine, bromine, $C_1$-$C_4$-alkyl or $C_2$-$C_4$-alkenyl, it being possible for each of these alkenyl radicals to be substituted by optionally substituted aryl, or represents phenyl which is optionally substituted by one to three identical or different substituents from the group comprising $C_1$-$C_4$-alkyl, halogen and/or halogeno$C_1$-$C_2$-alkyl with 1 to 5 halogen atoms, or |
| $R_1$ and $R_2$ | together represent a C-C bond, |
| $R_3$ | represents hydrogen, fluorine, chlorine, bromine, iodine or $C_1$-$C_{12}$-alkyl, or represents phenyl which is optionally substituted by one to three identical or different substituents from the group comprising $C_1$-$C_4$-alkyl, halogen and/or halogeno-$C_1$-$C_2$-alkyl with 1 to 5 halogen atoms, |
| $R_4$ | represents hydrogen, |
| $R_5$ | represents hydrogen or $C_1$-$C_4$-alkyl, |
| W | represents hydrogen, fluorine, chlorine or bromine, |
| X | represents nitrogen or a CH group and |
| Z | represents the groupings |

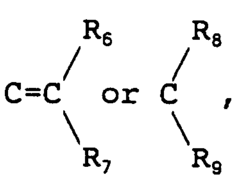

wherein

| | |
|---|---|
| $R_6$ | represents phenyl which is optionally substituted by one to three identical or different substituents from the group comprising $C_1$-$C_4$-alkyl, halogen and/or halogeno-$C_1$-$C_2$- |

alkyl with 1 to 5 halogen atoms, or

$R_6$ together with $R_4$ represents the grouping

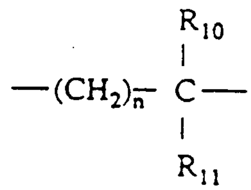

$R_7$ represents hydrogen or $C_1$-$C_4$-alkyl,

$R_8$ represents hydrogen or $C_1$-$C_4$-alkyl,

$R_9$ represents hydrogen or $C_1$-$C_4$-alkyl,

$R_{10}$ represents hydrogen or $C_1$-$C_4$-alkyl,

$R_{11}$ represents hydrogen or $C_1$-$C_4$-alkyl and

n represents zero or the numbers 1 or 2,

and of acid addition salts and metal salt complexes thereof, characterised in that phenol derivatives of the formula

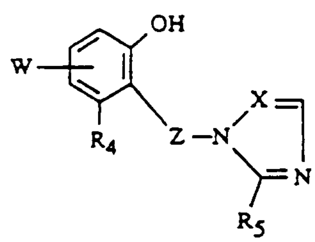

(II)

in which

$R_4$, $R_5$, W, X and Z have the abovementioned meaning,

are reacted with bases, if appropriate in the presence of a diluent, and the phenolates thereby formed, of the formula

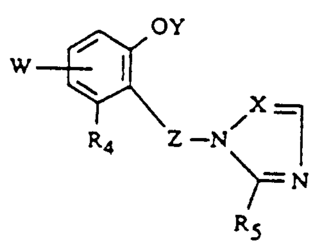

(IIa)

in which

$R_4$, $R_5$, W, X and Z have the abovementioned meaning and

Y represents a base radical,

are reacted with halogen compounds of the formula

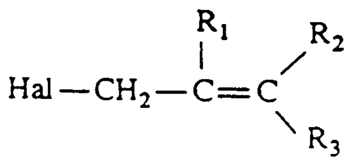

(III)

in which

$R_1$, $R_2$ and $R_3$ have the abovementioned meaning and

Hal represents chlorine, bromine or iodine,

in the presence of a diluent and if appropriate in the presence of an acid-binding agent, and, if appropriate, an acid or a metal salt is then added onto the compounds of the formula (I) thus obtained.

2. Microbicidal agents, characterised in that they contain at least one (azolyl-vinyl)-phenol alkenyl ether of the formula (I) according to Claim 1 or an acid addition salt or metal salt complex of an (azolyl-vinyl)-phenol alkenyl ether of the formula (I).

3. Use of (azolyl-vinyl)-phenol alkenyl ethers of the formula (I) according to Claim 1 or of acid addition salts and metal complexes thereof as microbicides in plant protection and the preservation of materials.

4. Method of combating undesirable microorganisms in plant protection and the preservation of materials, characterised in that (azolyl-vinyl)-phenol alkenyl ethers of the formula (I) according to Claim 1 or acid addition salts or metal salt complexes thereof are applied to the microorganisms and/or their environment.

5. Process for the preparation of microbicidal agents, characterised in that (azolyl-vinyl)-phenol alkenyl ethers of the formula (I) according to Claim 1 or acid addition salts or metal salt complexes thereof are mixed with extenders and/or surface-active substances.

**Revendications**

**Revendications pour les Etats contractants suivants : BE, CH, DE, FR, GB, GR, IT, LI, NL**

1. (Azolyl-vinyl)-phénol-alcényléthers de formule

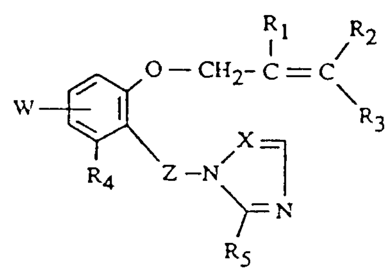

(I)

dans laquelle

$R_1$ représente l'hydrogène, le fluor, le chlore, le brome ou un reste alkyle en $C_1$ à $C_4$,

$R_2$ représente l'hydrogène, le fluor, le chlore, le brome, un reste alkyle en $C_1$ à $C_4$, alcényle en $C_2$ à $C_4$, chaque reste alcényle pouvant être substitué par un radical aryle éventuellement substitué, ou un groupe phényle portant éventuellement 1 à 3 substituants, identiques ou différents, alkyle en $C_1$ à $C_4$, halogéno et/ou halogénalkyle en $C_1$ ou $C_2$ ayant 1 à 5 atomes d'halogènes, ou

$R_1$ et $R_2$ forment conjointement une liaison carbone-à-carbone,

$R_3$ représente l'hydrogène, le fluor, le chlore, le brome, l'iode, un reste alkyle en $C_1$ à $C_{12}$ ou un reste phényle portant éventuellement 1 à 3 substituants, identiques ou différents, alkyle en $C_1$ à $C_4$, halogéno et/ou halogénalkyle en $C_1$ ou $C_2$ ayant 1 à 5 atomes

d'halogènes,

$R_4$ est l'hydrogène,

$R_5$ est l'hydrogène ou un groupe alkyle en $C_1$ à $C_4$,

W est l'hydrogène, le fluor, le chlore ou le brome,

X est l'azote ou un groupe CH et

Z représente les groupements

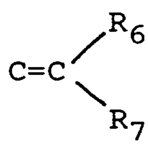 ou 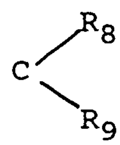 ,

où

$R_6$ est un groupe phényle portant facultativement 1 à 3 substituants, identiques ou différents, alkyle en $C_1$ à $C_4$, halogéno et/ou halogénalkyle en $C_1$ ou $C_2$ ayant 1 à 5 atomes d'halogènes, ou bien

$R_6$ forme conjointement avec $R_4$ le groupement

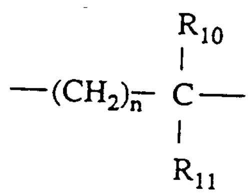

$R_7$ est l'hydrogène ou un groupe alkyle en $C_1$ à $C_4$,

$R_8$ est l'hydrogène ou un groupe alkyle en $C_1$ à $C_4$,

$R_9$ est l'hydrogène ou un groupe alkyle en $C_1$ à $C_4$,

$R_{10}$ est l'hydrogène ou un groupe alkyle en $C_1$ à $C_4$,

$R_{11}$ est l'hydrogène ou un groupe alkyle en $C_1$ à $C_4$,

n est égal à 0 ou aux nombres 1 ou 2,

ainsi que leurs sels d'addition d'acides et leurs complexes de sels métalliques.

**2.** (Azolyl-vinyl)-phénol-alcényléthers de formule (I) suivant la revendication 1, dans lesquels

$R_1$ représente l'hydrogène, le fluor, le chlore, le brome, un groupe méthyle, éthyle, n-propyle, isopropyle ou n-butyle,

$R_2$ est l'hydrogène, le fluor, le chlore, le brome, un groupe méthyle, éthyle, n-propyle, isopropyle, n-butyle, vinyle, propényle ou styryle, ou un groupe phényle portant facultativement 1 à 3 substituants, identiques ou différents, méthyle, éthyle, fluoro, chloro, bromo et/ou trifluorométhyle, ou bien

$R_1$ et $R_2$ forment ensemble une liaison carbone-à-carbone,

$R_3$ est l'hydrogène, le fluor, le chlore, le brome, l'iode, un groupe alkyle en $C_1$ à $C_{10}$ ou un groupe phényle portant facultativement 1 à 3 substituants, identiques ou différents, méthyle, éthyle, fluoro, chloro, bromo et/ou trifluorométhyle,

$R_4$ est l'hydrogène,

$R_5$ est l'hydrogène, un groupe méthyle ou éthyle,

W est l'hydrogène ou le chlore,

X est l'azote ou un groupe CH et

Z représente les groupements

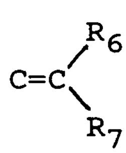 ou 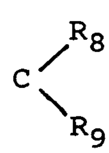 ,

où

| | |
|---|---|
| $R_6$ | est un groupe phényle portant facultativement 1 à 3 substituants, identiques ou différents, méthyle, éthyle, fluoro, chloro et/ou trifluorométhyle, |
| $R_6$ | forme conjointement avec $R_4$ le groupement |

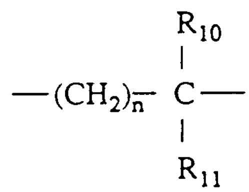

| | |
|---|---|
| $R_7$ | représente l'hydrogène, un groupe méthyle, éthyle, n-propyle, isopropyle ou n-butyle, |
| $R_8$ | est l'hydrogène, un groupe méthyle, éthyle, n-propyle, isopropyle ou n-butyle, |
| $R_9$ | est l'hydrogène, un groupe méthyle, éthyle, n-propyle, isopropyle ou n-butyle, |
| $R_{10}$ | est l'hydrogène ou le groupe méthyle, |
| $R_{11}$ | est l'hydrogène ou le groupe méthyle et |
| n | a la valeur 0 ou 1. |

**3.** Procédé de production de (azolyl-vinyl)-phénol-alkylène-éthers selon la revendication 1, ainsi que de leurs sels d'addition d'acides et de leurs complexes de sels métalliques, caractérisé en ce qu'on fait réagir des dérivés de phénol de formule

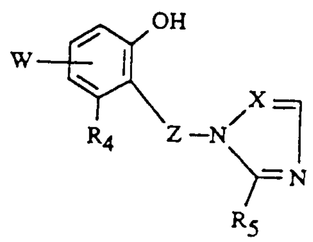

(II)

dans laquelle
$R_4$, $R_5$, W, X et Z ont la définition indiquée ci-dessus,
avec des bases, éventuellement en présence d'un diluant et on fait réagir les phénolates ainsi produits de formule

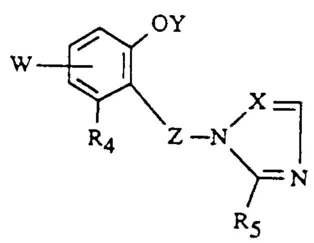

(IIa)

dans laquelle
$R_4$, $R_5$, W, X et Z ont la définition indiquée ci-dessus, et
Y représente un reste de base,
avec des composés halogénés de formule

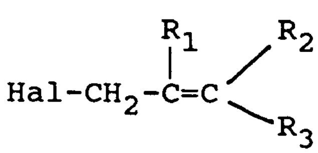

(III)

dans laquelle

$R_1$, $R_2$ et $R_3$ ont la définition indiquée ci-dessus, et

Hal représente le chlore, le brome ou l'iode,

en présence d'un diluant ainsi que, le cas échéant, en présence d'un accepteur d'acide et, le cas échéant, on additionne ensuite un acide ou un sel métallique sur les composés de formule (I) ainsi obtenus.

**4.** Compositions microbicides, caractérisées par une teneur en au moins un éther de (azolyl-vinyl)-phénol-alcényle de formule (I) suivant la revendication 1 ou d'un sel d'addition d'acide ou d'un complexe de sel métallique d'un (azolyl-vinyl)-phénol-alcényléther de formule (I).

**5.** Utilisation de (azolyl-vinyl)-phénol-alcényléthers de formule (I) suivant la revendication 1 ou de leurs sels d'addition d'acides et de leurs complexes de sels métalliques comme microbicides dans la protection des plantes et la protection de denrées.

**6.** Procédé pour combattre des micro-organismes indésirables dans la protection des plantes et dans la protection des denrées, caractérisé en ce qu'on fait agir des (azolyl-vinyl)-phénol-alcényléthers de formule (I) suivant la revendication 1 ou leurs sels d'addition d'acides ou leurs complexes de sels métalliques sur les micro-organismes et/ou sur leur milieu.

**7.** Procédé de préparation de compositions microbicides, caractérisé en ce qu'on mélange des (azolyl-vinyl)-phénol-alcényléthers de formule (I) suivant la revendication 1 ou leurs sels d'addition d'acides ou leurs complexes de sels métalliques avec des diluants et/ou des agents tensio-actifs.

**8.** (Azolyl-vinyl)-phénol-alcényléthers suivant la revendication 1, caractérisés par la formule

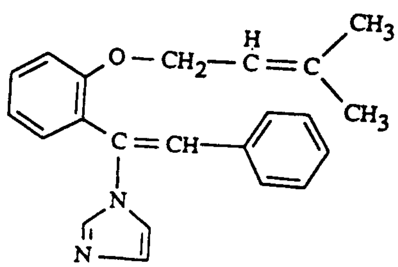

**9.** (Azolyl-vinyl)-phénol-alcényléthers suivant la revendication 1, caractérisés par la formule

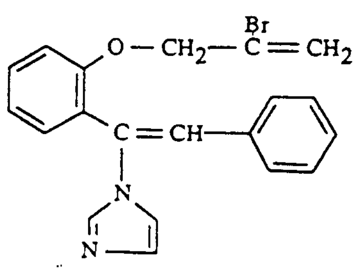

**Revendications pour l'Etat contractant suivant : ES**

**1.** Procédé de production de (azolyl-vinyl)-phénol-alcényléthers de formule

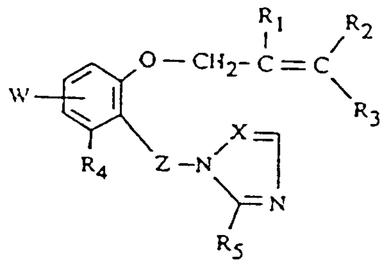

(I)

dans laquelle

| | |
|---|---|
| $R_1$ | représente l'hydrogène, le fluor, le chlore, le brome ou un reste alkyle en $C_1$ à $C_4$, |
| $R_2$ | représente l'hydrogène, le fluor, le chlore, le brome, un reste alkyle en $C_1$ à $C_4$, alcényle en $C_2$ à $C_4$, chaque reste alcényle pouvant être substitué par un radical aryle éventuellement substitué, ou un groupe phényle portant éventuellement 1 à 3 substituants, identiques ou différents, alkyle en $C_1$ à $C_4$, halogéno et/ou halogénalkyle en $C_1$ ou $C_2$ ayant 1 à 5 atomes d'halogènes, ou |
| $R_1$ et $R_2$ | forment conjointement une liaison carbone-à-carbone, |
| $R_3$ | représente l'hydrogène, le fluor, le chlore, le brome, l'iode, un reste alkyle en $C_1$ à $C_{12}$ ou un reste phényle portant éventuellement 1 à 3 substituants, identiques ou différents, alkyle en $C_1$ à $C_4$, halogéno et/ou halogénalkyle en $C_1$ ou $C_2$ ayant 1 à 5 atomes d'halogènes, |
| $R_4$ | est l'hydrogène, |
| $R_5$ | est l'hydrogène ou un groupe alkyle en $C_1$ à $C_4$, |
| W | est l'hydrogène, le fluor, le chlore ou le brome, |
| X | est l'azote ou un groupe CH et |
| Z | représente les groupements |

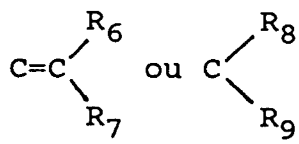

,

| | |
|---|---|
| | où |
| $R_6$ | est un groupe phényle portant facultativement 1 à 3 substituants, identiques ou différents, alkyle en $C_1$ à $C_4$, halogéno et/ou halogénalkyle en $C_1$ ou $C_2$ ayant 1 à 5 atomes d'halogènes, ou bien |
| $R_6$ | forme conjointement avec $R_4$ le groupement |

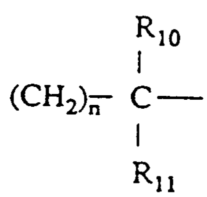

| | |
|---|---|
| $R_7$ | est l'hydrogène ou un groupe alkyle en $C_1$ à $C_4$, |
| $R_8$ | est l'hydrogène ou un groupe alkyle en $C_1$ à $C_4$, |
| $R_9$ | est l'hydrogène ou un groupe alkyle en $C_1$ à $C_4$, |
| $R_{10}$ | est l'hydrogène ou un groupe alkyle en $C_1$ à $C_4$, |
| $R_{11}$ | est l'hydrogène ou un groupe alkyle en $C_1$ à $C_4$, |
| n | est égal à 0 ou aux nombres 1 ou 2, |

ainsi que de leurs sels d'addition d'acides et de leurs complexes de sels métalliques, caractérisé en ce qu'on fait réagir des dérivés phénoliques de formule

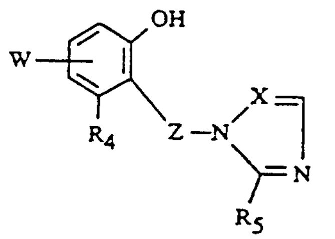

(II)

dans laquelle
$R_4$, $R_5$, W, X et Z ont la définition indiquée ci-dessus,
avec des bases, éventuellement en présence d'un diluant, et on fait réagir les phénolates ainsi produits de formule

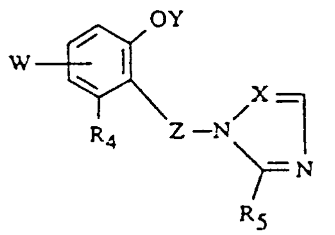

(IIa)

dans laquelle
$R_4$, $R_5$, W, X et Z ont la définition indiquée ci-dessus, et
Y représente un reste de base,
avec des composés halogénés de formule

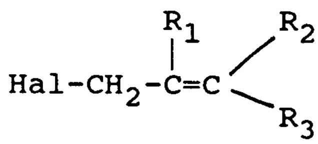

(III)

dans laquelle
$R_1$, $R_2$ et $R_3$ ont la définition indiquée ci-dessus, et
Hal représente le chlore, le brome ou l'iode,
en présence d'un diluant ainsi que, le cas échéant, en présence d'un accepteur d'acide et on additionne ensuite éventuellement un acide ou un sel métallique sur les composés de formule (I) ainsi obtenus.

**2.** Compositions microbicides, caractérisées par une teneur en au moins un (azolyl-vinyl)-phénol-alcényléther de formule (I) suivant la revendication 1 ou en un sel d'addition d'acide ou en un complexe de sel métallique d'un (azolyl-vinyl)-phénol-alcényléther de formule (I).

**3.** Utilisation de (azolyl-vinyl)-phénol-alcényléthers de formule (I) suivant la revendication 1 ou de leurs sels d'addition d'acides et de leurs complexes métalliques comme microbicides dans la protection des plantes et dans la protection des denrées.

**4.** Procédé pour combattre des micro-organismes indésirables dans la protection des plantes et dans la protection des denrées, caractérisé en ce qu'on fait agir des (azolyl-vinyl)-phénol-alcényléthers de formule (I) suivant la revendication 1 ou leurs sels d'addition d'acides ou leurs complexes de sels métalliques sur les micro-organismes et/ou sur leur milieu.

**5.** Procédé de préparation de compositions microbicides, caractérisé en ce qu'on mélange des (azolyl-vinyl)-phénol-alcényléthers de formule (I) suivant la revendication 1 ou leurs sels d'addition d'acides ou

leurs complexes de sels métalliques avec des diluants et/ou des agents tensio-actifs.